(19) Europäisches Patentamt
European Patent Office
Office européen des brevets

(11) EP 1 172 090 B1

(12) **FASCICULE DE BREVET EUROPEEN**

(45) Date de publication et mention de la délivrance du brevet:
**09.03.2005 Bulletin 2005/10**

(51) Int Cl.7: **A61K 7/48**

(21) Numéro de dépôt: **01401691.9**

(22) Date de dépôt: **25.06.2001**

(54) **Compositions pour la coloration de la peau comprenant la dihydroxyacétone et un sel de flavylium non substitué en position 3**

Hautfärbemittel enthaltend Dihydroxyaceton und einen in Position 3 unsubstituierte Flavylium-Salz Verbindung

Skin dyeing compositions comprising dihydroxyacetone and a flavylium salt non substituted in position 3

(84) Etats contractants désignés:
**AT BE CH CY DE DK ES FI FR GB GR IE IT LI LU MC NL PT SE TR**

(30) Priorité: **12.07.2000 FR 0009118**

(43) Date de publication de la demande:
**16.01.2002 Bulletin 2002/03**

(73) Titulaire: **L'OREAL**
**75008 Paris (FR)**

(72) Inventeurs:
- **Candau, Didier**
  **91570 Bievres (FR)**
- **Forestier, Serge**
  **77410 Claye Souilly (FR)**

(74) Mandataire: **Miszputen, Laurent**
**L'OREAL - D.I.P.I.**
**25-29 Quai Aulagnier**
**92600 Asnières (FR)**

(56) Documents cités:
**FR-A- 2 757 383**

- **DATABASE WPI Week 199929 Derwent Publications Ltd., London, GB; AN 1999-338248 XP002164093 & CN 1 209 992 A (ZHANG)**
- **DATABASE WPI Week 198828 Derwent Publications Ltd., London, GB; AN 1988-195805 XP002164094 & JP 63 135310 A (LION CORP)**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 172 090 B1

Printed by Jouve, 75001 PARIS (FR)

## Description

La présente invention se rapporte à des compositions cosmétiques et/ou dermatologiques destinées à la coloration artificielle de la peau, caractérisée par le fait qu'elle comprend, dans un support cosmétiquement acceptable, la dihydroxyacétone (DHA) et au moins un composé du type sel de flavylium, non substitué en position 3, et substitué par au moins un radical hydroxyle ou alcoxy, obtenu par voie de synthèse ou à partir d'un extrait végétal le contenant ou bien encore à partir d'un extrait végétal enrichi le contenant.

L'invention concerne également les applications de ces compositions la coloration de la peau.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de composés mono ou polycarbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. De plus, la coloration produite sur la peau par la DHA est souvent jugée trop jaune par les utilisateurs.

Ainsi, on est toujours à la recherche de nouveaux composés et de nouvelles compositions permettant de conférer artificiellement à la peau une coloration proche du bronzage naturel d'une manière simple, efficace, rapide et sans risque.

Les colorants anthocyaniques sont connus depuis longtemps comme colorants alimentaires et pharmaceutiques. Ces anthocyanes sont présents dans la nature sous forme d'hétérosides appelés anthocyanosides et de génines, appelées anthocyanidines. Ces anthocyanes sont des dérivés du phényl-2-benzopyrylium ou flavylium et sont notamment présents dans la plante sous forme de sels. Les anthocyanes sont des composés de teinte rouge, violette ou bleue qui colorent généralement les fleurs, les fruits et parfois les feuilles. La couleur observée dépend à la fois de la structure de la génine majoritaire et des conditions du milieu où se trouvent les colorants anthocyaniques.

Or, à la suite d'importantes recherches menées dans le domaine de la coloration artificielle de la peau, la Demanderesse a maintenant découvert que l'association de la DHA et d'un ou plusieurs composés particuliers du type sel de flavylium non substitué en position 3 permettait de conférer immédiatement après l'application sur la peau du produit une coloration artificielle plus proche du bronzage naturel. En effet, la demanderesse a constaté que ladite association permettait d'obtenir par rapport à un autobronzant du type carbonylé tel que la DHA utilisé seul, une montée de couleur sur la peau dans un temps beaucoup plus court (par exemple au bout de 30 minutes). De plus, ladite association permet d'obtenir une nuance proche du bronzage naturel et stable dans le temps.

La présente invention a donc pour objet une nouvelle composition cosmétique et/ou dermatologique, destinée à la coloration artificielle de la peau proche du bronzage naturel, caractérisée par le fait qu'elle comprend, dans un support cosmétiquement acceptable, la DHA et au moins un composé du type sel de flavylium, non substitué en position 3, et substitué par au moins un radical hydroxyle ou alcoxy, obtenu par voie de synthèse ou à partir d'un extrait végétal le contenant ou bien encore à partir d'un extrait végétai enrichi.

La présente invention a encore pour objet l'utilisation nouvelle de l'association de DHA et d'au moins un composé du type sel de flavylium, non substitué en position 3, et substitué par au moins un radical hydroxyle ou alcoxy, obtenu par voie de synthèse, ou à partir d'un extrait végétal, ou d'un extrait végétal enrichi, dans des compositions cosmétiques et/ou dermatologiques dans le but de conférer à la peau une coloration artificielle proche du bronzage naturel.

La présente invention a également pour objet un procédé de coloration artificielle proche du bronzage naturel de la peau, caractérisé en ce qu'il consiste à appliquer sur celle-ci une quantité efficace de l'association de DHA et d'au moins un composé du type sel de flavylium, non substitué en position 3, et substitué par au moins un radical hydroxyle ou alcoxy, obtenu par voie de synthèse, ou à partir d'un extrait végétal, ou d'un extrait végétal enrichi dans des compositions cosmétiques .

Les compositions et les utilisations conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel.

Au sens de la présente invention, on entendra, par « composition destinée à la coloration artificielle de la peau », une formulation ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

D'autres caractéristiques, aspects et avantages de la présente invention apparaîtront à la lecture de la description détaillée qui va suivre.

Les compositions conformes à la présente invention conduisent en général au bout de 30 minutes après application sur une peau claire à raison de $2mg/cm^2$ à un assombrissement caractérisé dans le système de mesure colorimètrique (L*, a*, b*) par un $\Delta L^*$ allant de -0,5 à - 20. De façon plus préférentielle, $\Delta L^*$ variera de - 0,5 à - 15.

Les compositions conformes à la présente invention apportent au bout de 30 minutes après application sur la peau à raison de $2mg/cm^2$ une coloration sur une peau claire qui est définie dans le système de mesure colorimétrique (L*, a*, b*), par un rapport $\Delta a^*/\Delta b^*$ allant de 0,5 à 3 et encore plus particulièrement de 0, 8 à 2.

Selon la présente invention, on entend « par peau claire », des peaux non bronzées dont on peut définir les caractéristiques colorimétriques par leur angle ITA tel que défini dans la publication de A. Chardon et al. « Skin Colour Typology and Suntanning Pathways » et présentée au $16^{th}$ IFSCC congress oct 8-10 1990 de New-York, et in *Int. J. Cosm. Sci.***13** 191-208 (1991). Les peaux claires telles que définies dans cette classification ont un angle ITA compris entre 35 et 55.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.

$\Delta L^*$ traduit l'assombrissement de la couleur : plus le $\Delta L^*$ est négatif, plus la couleur s'est assombrie avec :

$$\Delta L^* = L^* \text{ peau non colorée} - L^* \text{ peau colorée}$$

Le rapport $\Delta a^*/\Delta b^*$ traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Parmi les composés du type sel de flavylium non substitués en position 3 conformes à l'invention, on utilisera de préférence ceux répondant à la formule (I) suivante :

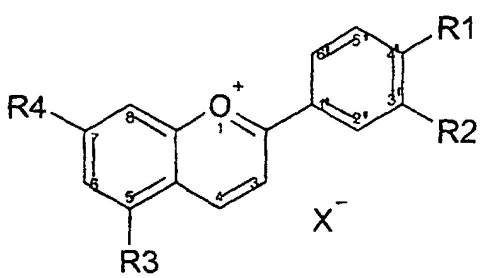

(I)

formule (I) dans laquelle :

- $R_1$ désigne un radical OH ou alcoxy en $C_1$-$C_8$, linéaire ou ramifié, saturé ou insaturé,
- $R_2$, $R_3$, $R_4$, identiques ou différents, désignent H ou $R_1$, étant entendu qu'au moins un des radicaux $R_1$ à $R_4$ désigne OH,
- $X^-$ est un anion organique ou minéral et de préférence un dérivé d'acide minéral tel que par exemple un halogénure comme bromure ou chlorure ou bien un dérivé d'un acide organique comme par exemple acétate, borate, citrate, tartrate, lactate, bisulfate, sulfate, phosphate.

Les composés de formule (I) particulièrement préférés selon la présente invention sont choisis dans le groupe de ceux pour lesquels, dans la formule (I), $R_1$ désigne OH ou $OCH_3$.

On peut citer notamment parmi eux, les chlorures des composés suivants :

- 4', 5, 7-trihydroxyflavylium, communément dénommé "chlorure d'apigéninidine",
- 3', 4', 7- trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium,
- 3',4', 5, 7-tétrahydroxyflavylium,
- 3', 4', 5', 5, 7-pentahydroxyflavylium.

Parmi ces composés, le chlorure d'apigéninidine (chlorure de 4', 5, 7-trihydroxyflavylium) et le chlorure de 3', 4', 7- trihydroxyflavylium sont encore plus particulièrement préférés.

Une forme particulière de l'invention consiste à utiliser le chlorure d'apigéninidine sous forme d'un extrait végétal, aisément préparé par extraction, et isolé, à partir de feuilles de Sorghum caudatum selon les procédés décrits dans les brevets CN 1064284A et CN1035512C ou toutes autres variantes de ces procédés .

Il peut aussi être extrait des tiges, des graines, ou des feuilles de Sorghum Bicolor, des pétales de Gesneria Fulgens, ainsi que des espèces Blechum Procerum et Sorgho en association avec du Colletotrichum Graminicola.

Une forme particulièrement préférée de l'invention est un extrait provenant des feuilles de Sorghum Bicolor obtenu par une extraction hydro-alcoolique en milieu acide à une température d'extraction allant de 30 à 40°C avec un rapport volume de solvant / masse de feuilles de Sorghum Bicolor allant de 10 à 30. Ledit extrait végétal de Sorghum titre environ 0,05 à 50% en poids de chlorure d'apigéninidine.

Les composés du type sel de flavylium, non substitués en position 3, et substitués par au moins un radical hydroxyle ou alcoxy, selon l'invention, peuvent être facilement obtenus par voie de synthèse, et à faible coût, notamment par la méthode bien connue de R. Robinson et D. Pratt J. Chem. Soc. 745 (1923). Ladite méthode implique de condenser une orthohydroxybenzaldéhyde ou ses dérivés de substitution sur une acétophénone ou ses dérivés de substitution, pour obtenir, en choisissant les substituants, les composés de formule (I) désirés.

En prenant comme exemple le chlorure d'apigéninidine (chlorure de 4', 5, 7-trihydroxyflavylium), le schéma de synthèse (i) peut être le suivant :

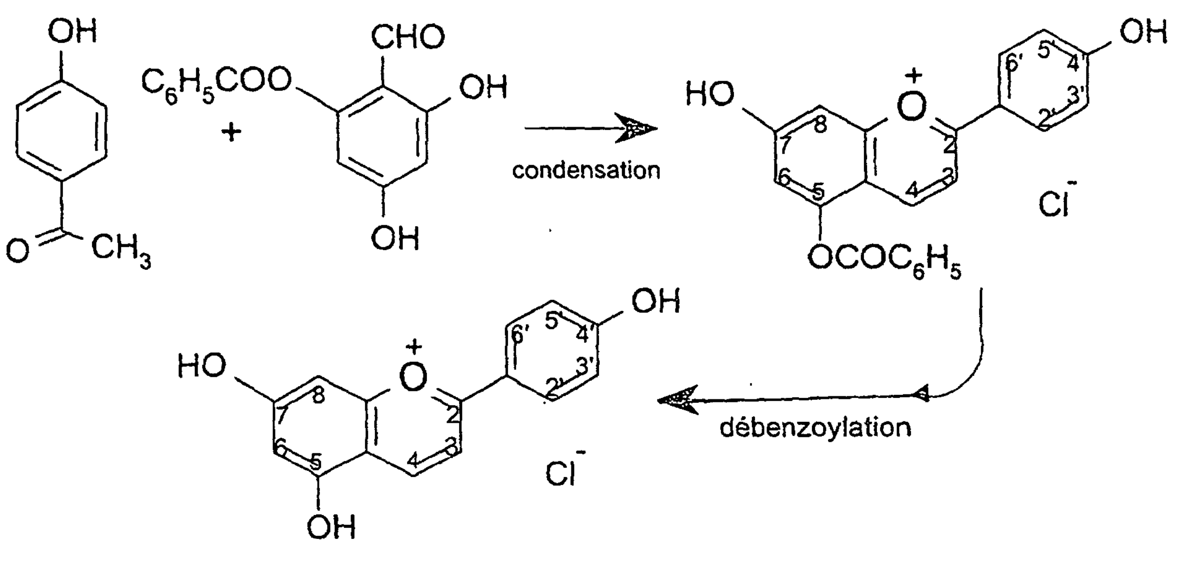


En prenant comme exemple le chlorure de 3', 4', 7- trihydroxyflavylium, le schéma de synthèse (ii) peut être le suivant :

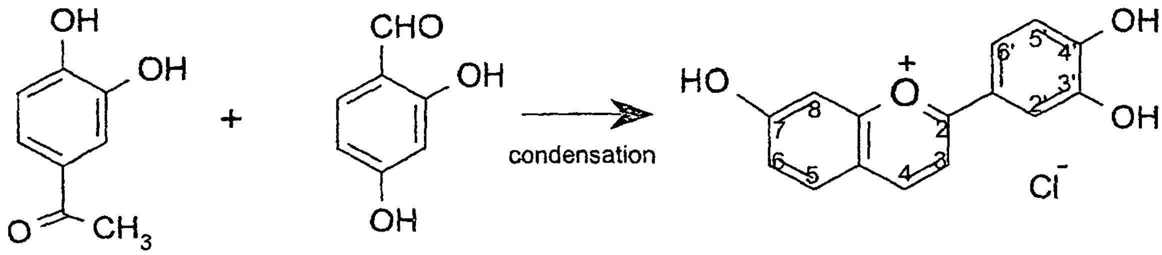


Diverses voies de synthèses, bien connues dans l'art antérieur, conduisent à l'apigéninidine.

Une méthode consiste, par exemple, à préparer, dans une première étape, la triméthylapigéninidine, par condensation du 4,6-diméthoxy-2-hydroxybenzaldéhyde commercial sur la 4-méthoxyacétophénone commerciale à 0°C en milieu éther anhydre, et saturation par HCl anhydre, pour obtenir après filtration un précipité rouge-orangé de triméthylapigéninidine. Dans une seconde étape, à hydrolyser la triméthylapigéninidine obtenue à l'étape précédente en chlorure d'apigéninidine, la réaction s'effectuant en milieu HI et phénol et AgCl en solution dans le méthanol. Une telle méthode de synthèse est décrite par R. Robinson et A. Robertson dans J. Chem. Soc. 1951 (1926) et 2196 (1927).

Une autre méthode consiste à condenser le 2, 4, 6-trihydroxybenzaldéhyde sur la 4-hydroxyacétophénone à 0°C en milieu solvant anhydre (acétate d'éthyle par exemple), et saturation par HCl anhydre, pour obtenir le chlorure d'apigéninidine. Une telle méthode est décrite par R. Robinson et A. Robertson dans J. Chem. Soc. 1528 (1928).

Une autre méthode de préparation du chlorure d'apigéninidine consiste à réduire une flavone, la naringénine, ou son dérivé triacétylé, par $NaBH_4$, puis à oxyder le produit obtenu par le chloranil (tétrachloro-1,4-benzoquinone). Ladite méthode est décrite par J. G. Sweeny et G. A. Iacobucci dans la revue Tetrahedron **33** 2923 -2927 (1977).

La méthode la plus particulièrement préférée, selon la présente invention, consiste à condenser le 2,4-dihydroxy-6-benzoyloxybenzaldéhyde sur la 4-hydroxyacétophénone à 0°C en milieu acétate d'éthyle anhydre, à saturer par HCl anhydre, puis à débenzoyler le produit obtenu par la soude, afin d'obtenir le chlorure d'apigéninidine avec un rendement élevé, suivant le schéma (i) décrit ci-dessus. Ladite méthode est décrite par R. Robinson et J. C. Bell dans J. Chem. Soc. 813 (1934).

La concentration en composé du type sel de flavylium, tel que décrit selon la présente invention, varie de préférence de environ 0,0001 à 10%, et encore plus préférentiellement de 0,001 à 5% en poids, par rapport au poids total de la composition.

La DHA est généralement présente dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les parfums, les conservateurs, les tensioactifs, les charges, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en $C_{12}C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Selon une forme particulièrement préférée, les compositions selon l'invention contiennent au moins 5% en poids par rapport au poids de la composition d'un ou plusieurs solvants polyhydroxylés. Ces derniers peuvent être choisis parmi les glycols et les éthers de glycol comme l'éthylène glycol, le propylène glycol, le butylène glycol, le dipropylène glycol ou le diéthylène glycol. Et plus particulièrement, les compositions selon l'invention contiennent un

mélange d'au moins trois solvants polyhydroxylés différents et encore plus particulièrement un mélange constitué de propylène glycol, de butylène glycol, et de dipropylène glycol.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. **13**, 238 (1965), FR 2 315 991 et FR 2 416 008).

Des exemples concrets, mais nullement limitatifs, illustrant l'invention, vont maintenant être donnés.

**<u>EXEMPLE 1 :</u>**

On prépare un extrait de Sorghum Bicolor titrant à 20-30% de chlorure d'apigéninidine selon le procédé de préparation suivant :

un extrait provenant des feuilles de Sorghum Bicolor est obtenu par extraction hydro-alcoolique (éthanol 95°) en milieu acide (0.2% HCl) à une température d'extraction de 35°C avec un rapport volume de solvant / masse de feuilles de Sorghum Bicolor de 15. L'extrait végétal de Sorghum est séché à l'étuve 24 h à 40°C et tamisé à 200 µm.

Le rendement de cette extraction est de 22.42% en matière colorante.
Le titre de l'extrait ainsi obtenu est de 21% en poids de chlorure d'apigéninidine.

Cet exemple vise à montrer l'intensité de la coloration obtenue avec un extrait de Sorghum Bicolor associé à la DHA conforme à la présente invention ainsi que la rapidité avec laquelle cette coloration se développe par rapport à une composition contenant la DHA seule à titre d'agent de coloration de la peau.

Cet exemple vise à montrer que la coloration obtenue par l'association de la DHA avec un extrait de Sorghum Bicolor conforme à la présente invention permet d'obtenir une intensité de couleur élevée immédiatement après application et stable dans le temps.

La Demanderesse a réalisé les compositions suivantes (les quantités sont exprimées en pourcentage de poids par rapport au poids total de la composition) :

<u>Composition A (hors invention):</u>

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Dihydroxyacétone (DHA) 4g
- Chlorure de sodium 2g
- Propylène glycol 23g
- Butylène glycol 5g
- Dipropylène glycol 10g
- Citrate trisodique 0.542g
- Acide citrique 0.209g
- Eau déminéralisée 32.649g

Composition B (invention)

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1 g
- Eau déminéralisée 32.55g
- Chlorure de sodium 2g
- Propylène glycol 23g
- Butylène glycol 5g
- Dipropylène glycol 10g
- Extrait de Sorghum Bicolor tel que préparé ci-dessus 0.1 g
- Dihydroxyacétone 4g
- Citrate trisodique 0.542g
- Acide citrique 0.208g

**Protocole d'évaluation :**

Les compositions A et B ont été appliquées à raison de 2 $mg/cm^2$ sur une zone de 7 x 4,5 $cm^2$ délimitée sur le dos de 6. volontaires dont la couleur de peau caractérisée par l'angle ITA est compris entre 35 et 55.

Les cinq séries de mesures colorimétriques suivantes ont été effectuées à l'aide d'un colorimètre Minolta CR-300 :

- 1°) avant application de la composition,
- 2°) 30 minutes après l'application,
- 3°) 2 heures après application.
- 4°) 4 heures après application.

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^* \text{ peau non colorée} - L^* \text{ peau colorée}$$

Pour la nuance de la coloration obtenue, on s'intéresse au rapport Δa*/Δb* qui traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Les résultats obtenus sont rassemblés dans le tableau (I) suivant :

Tableau (I) :

| | **Composition A (comparative) ΔL*** | **Composition B (invention) ΔL*** | **Composition B (invention) Δa*/Δb*** |
|---|---|---|---|
| 30 minutes | -0.4 | -1.6 | 0.8 |
| 2 heures | -1.1 | -2.0 | 0.7 |
| 4 heures | -2.5 | -2.7 | 0.9 |

On constate ainsi que 30 minutes après l'application, la composition B contenant la DHA et l'extrait végétal colorant permet d'obtenir un assombrissement très proche de celui obtenu avec la composition A contenant uniquement la DHA au bout de 4 heures.

La composition B contenant la DHA et l'extrait végétal colorant permet également d'obtenir une nuance proche du bronzage naturel et constante dans le temps

## Revendications

1. Composition cosmétique et/ou dermatologique pour la coloration artificielle de la peau proche du bronzage naturel, **caractérisée par le fait qu'**elle comprend, dans un support cosmétiquement acceptable, **la dihydroxyacétone (DHA)** et au moins un composé du type sel de flavylium, non substitué en position 3, et substitué par au moins un radical hydroxyle ou alcoxy qui répond à la formule (I) suivante :

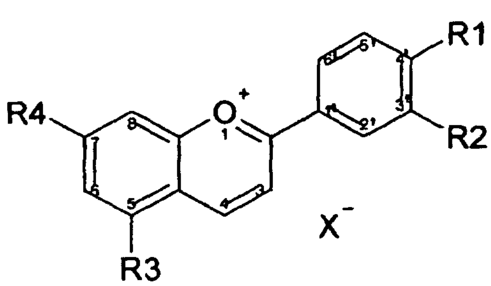

(I)

dans laquelle :

- $R_1$ désigne un radical OH ou alcoxy en $C_1$-$C_8$, linéaire ou ramifié, saturé ou insaturé,
- $R_2$, $R_3$, $R_4$, identiques ou différents, désignent H ou $R_1$,
  étant entendu qu'au moins un des radicaux $R_1$ à $R_4$ désigne OH,
- $X^-$ est un anion organique ou minéral.

obtenu par voie de synthèse ou à partir d'un extrait végétal le contenant ou bien encore à partir d'un extrait végétal enrichi.

2. Composition selon la revendication 1, **caractérisée par le fait qu'**elle conduit au bout de 30 minutes après application sur une peau claire à raison de 2mg/cm2 à un assombrissement caractérisé dans le système de mesure colorimétrique L* a* b* par un ΔL* allant de - 0,5 à -20.

3. Composition selon la revendication 2, où le ΔL* varie de - 0,5 à -15.

4. Composition selon la revendication 1 à 3, **caractérisée par le fait qu'**elle produit au bout de 30 minutes après application sur une peau claire à raison de $2mg/cm^2$ une coloration définie dans le système colorimétrique L* a* b* par un rapport Δa*/Δb* allant de 0,5 à 3.

5. Composition selon la revendication 4, selon laquelle le rapport Δa*/Δb* varie de 0,8 à 2.

6. Composition selon la revendication 1, où dans la formule (I), $X^-$ est un halogénure ou un dérivé d'un acide organique.

7. Composition selon la revendication 1, où dans la formule (1), $R_1$ désigne OH ou $OCH_3$.

8. Composition selon la revendication 1, où le composé de formule (I) est choisi parmi les chlorures des composés suivants :

- 4', 5, 7-trihydroxyflavylium (chlorure d'apigéninidine),
- 3', 4', 7- trihydroxyflavylium,
- 4'-hydroxyflavylium,

- 4', 7-dihydroxyflavylium,
- 3', 4'-dihydroxyflavylium,
- 3', 4'-dihydroxy-7-méthoxy-flavylium.
- 3',4', 5, 7-tétrahydroxyflavinium,
- 3', 4', 5', 5, 7-pentahydroxyflavinum.

**9.** Composition selon la revendication 8, **caractérisée par le fait qu'**il s'agit du chlorure de 4', 5, 7-trihydroxyflavylium sous forme pure susceptible d'être obtenu par voie de synthèse.

**10.** Composition selon la revendication 8, **caractérisée par le fait qu'**il s'agit du chlorure de 4', 5, 7-trihydroxyflavylium, sous forme d'extrait végétal.

**11.** Composition selon la revendication 10, **caractérisée par le fait que** l'extrait végétal est un extrait végétal, obtenu à partir de feuilles de Sorghum caudatum, ; de tiges, de graines ou de feuilles de Sorghum Bicolor ; des pétales de Gesneria Fulgens, ainsi que des espèces Blechum Procerum et Sorgho en association avec du Colletotrichum Graminicola.

**12.** Composition selon la revendication 10, **caractérisée par le fait que** l'extrait végétal est un extrait de Sorghum Bicolor susceptible d'être obtenu par une extraction hydro-alcoolique acide à une température d'extraction allant de 30 à 40°C avec un rapport volume de solvant / masse de feuilles de Sorghum Bicolor allant de 10 à 30.

**13.** Composition selon la revendication 12, **caractérisée par le fait que** l'extrait de Sorghum Bicolor titre de 0,05 à 50% en poids de chlorure de 4', 5, 7-trihydroxy sel de flavylium.

**14.** Composition selon l'une quelconque des revendications 1 à 13, où la concentration en composé du type sel de flavylium varie de 0,0001 à 10% par rapport au poids total de la composition.

**15.** Composition selon la revendication 14, où la concentration en composé du type sel de flavylium, varie de 0,001 à 5% en poids, par rapport au poids total de la composition.

**16.** Composition selon l'une quelconque des revendications 1 à 15, où **la dihydroxyacétone** est présente dans des proportions allant de 0,1 à 10% en poids par rapport au poids total de la composition.

**17.** Composition selon l'une quelconque des revendications 1 à 16, contenant au moins 5% en poids par rapport au poids de la composition d'un ou plusieurs solvants polyhydroxylés.

**18.** Composition selon la revendication 17, où les solvants polyhydroxylés sont choisis parmi les glycols et les éthers de glycol.

**19.** Composition selon la revendication 17 ou 18, où les solvants polyhydroxylés sont choisis parmi l'éthylène glycol, le propylène glycol, le butylène glycol, le dipropylène glycol ou le diéthylène glycol et leurs mélanges.

**20.** Composition selon l'une quelconque des revendications 17 à 19, contenant un mélange de trois solvants polyhydroxylés différents .

**21.** Composition selon la revendication 20, contenant un mélange constitué de propylène glycol, de butylène glycol, et de dipropylène glycol.

**22.** Procédé de traitement cosmétique de la peau destiné à lui conférer une coloration artificielle proche du bronzage naturel, **caractérisé en ce qu'**il consiste à appliquer sur celle-ci une quantité efficace d'une composition tels que défini à l'une quelconque des revendications 1 à 21.

**23.** Utilisation **de la dihydroxyacétone (DHA)** et d'au moins un composé du type sel de flavylium tel que défini à l'une quelconque des revendications précédentes dans des, ou pour la fabrication de, compositions cosmétiques et/ou dermatologiques dans le but de conférer à la peau une coloration artificielle proche du bronzage naturel.

## Patentansprüche

1. Kosmetische und/oder dermatologische Zusammensetzung zur künstlichen Färbung der Haut in einer Farbnuance, die der natürlichen Bräunung nahe kommt, **dadurch gekennzeichnet, dass** sie in einem kosmetisch akzeptablen Träger Dihydroxyaceton (DHA) und mindestens eine Verbindung vom Typ der Flavyliumsalze der folgenden Formel (I) enthält, die in 3-Stellung nicht substituiert und mit mindestens einer Hydroxygruppe oder Alkoxygruppe substituiert ist und die auf synthetischem Wege hergestellt oder aus einem Pflanzenextrakt oder einem angereicherten Pflanzenextrakt, der sie enthält, gewonnen wird:

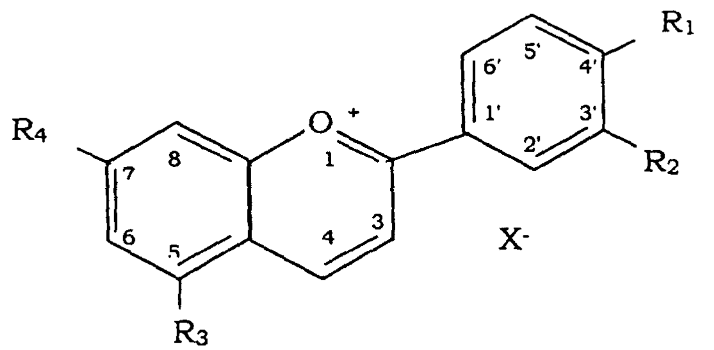

(I)

worin bedeuten:

- $R_1$ die OH-Gruppe oder eine geradkettige oder verzweigte, gesättigte oder ungesättigte $C_{1-8}$-Alkoxygruppe,
- $R_2$, $R_3$ und $R_4$, die gleich oder verschieden sind, H oder $R_1$, mit der Maßgabe, dass mindestens eine der Gruppen $R_1$ bis $R_4$ OH bedeutet,
- $X^-$ ein organisches oder anorganisches Anion.

2. Zusammensetzung nach Anspruch 1, **dadurch gekennzeichnet, dass** sie 30 Minuten nach dem Auftragen in einer Menge von 2 $mg/cm^2$ auf helle Haut ein Dunklerwerden der Hautfarbe bewirkt, das im colorimetrischen L* a* b*-System durch ein $\Delta L^*$ von -0,5 bis -20 gekennzeichnet ist.

3. Zusammensetzung nach Anspruch 2, **dadurch gekennzeichnet, dass** $\Delta L^*$ im Bereich von -0,5 bis -15 liegt.

4. Zusammensetzung nach einem der Ansprüche 1 bis 3, **dadurch gekennzeichnet, dass** sie 30 Minuten nach dem Auftragen auf einen hellen Hauttyp in einer Menge von 2 mg/ $cm^2$ zu einer Färbung führt, die im colorimetrischen System L*, a*, b* durch ein Verhältnis $\Delta a^*/\Delta b^*$ von 0,5 bis 3 definiert ist.

5. Zusammensetzung nach Anspruch 4, **dadurch gekennzeichnet, dass** das Verhältnis $\Delta a^*/\Delta b^*$ im Bereich von 0,8 bis 2 liegt.

6. Zusammensetzung nach Anspruch 1, wobei in der Formel (I) $X^-$ ein Halogenid oder ein Derivat einer organischen Säure bedeutet bedeutet.

7. Zusammensetzung nach Anspruch 1, wobei in der Formel (1) $R_1$ OH oder $OCH_3$ bedeutet.

8. Zusammensetzung nach Anspruch 1, wobei die Verbindung der Formel (1) unter den folgenden Chloriden ausgewählt ist:

- 4',5,7-Trihydroxyflavyliumchlorid (oder Apigeninidinchlorid),
- 3',4',7-Trihydroxyflavyliumchlorid,
- 4'-Hydroxyflavyliumchlorid,
- 4',7-Dihydroxyflavyliumchlorid,
- 3',4'-Dihydroxyflavyliumchlorid,
- 3',4'-Dihydroxy-7-methoxyflavyliumchlorid,
- 3',4',5,7-Tetrahydroxyflavyliumchlorid,

- 3',4',5',5,7-Pentahydroxyflavyliumchlorid.

**9.** Zusammensetzung nach Anspruch 8, **dadurch gekennzeichnet, dass** es sich um das 4',5,7-Trihydroxyflavyliumchlorid in reiner Form handelt, das auf synthetischem Wege erhältlich ist.

**10.** Zusammensetzung nach Anspruch 8, **dadurch gekennzeichnet, dass** es sich um das 4',5,7-Trihydroxyflavyliumchlorid in Form eines Pflanzenextrakts handelt.

**11.** Zusammensetzung nach Anspruch 10, **dadurch gekennzeichnet, dass** der Pflanzenextrakt ein Pflanzenextrakt ist, der aus den Blättern von Sorghum caudatum; Stängeln, Samen oder Blättern von Sorghum Bicolor; Blütenblättern von Gesneria Fulgens sowie den Arten Blechum Procerum und Sorgho in Kombination mit Colletotrichum Graminicola erhalten wird.

**12.** Zusammensetzung nach Anspruch 10, **dadurch gekennzeichnet, dass** der Pflanzenextrakt ein Extrakt von Sorghum Bicolor ist, der durch eine wässerig-alkoholische saure Extraktion bei einer Extraktionstemperatur von 30 bis 40 °C mit einem Volumenverhältnis Lösungsmittel/Masse der Blätter von Sorghum Bicolor von 10 bis 30 erhältlich ist.

**13.** Zusammensetzung nach Anspruch 12, **dadurch gekennzeichnet, dass** der Extrakt von Sorghum Bicolor 0,05 bis 50 Gew.-% 4',5,7-Trihydroxyflavyliumchlorid enthält.

**14.** Zusammensetzung nach einem der Ansprüche 1 bis 12, wobei die Konzentration der Verbindung vom Typ der Flavyliumsalze im Bereich von 0,0001 bis 10 %, bezogen auf das Gesamtgewicht der Zusammensetzung, liegt.

**15.** Zusammensetzung nach Anspruch 14, wobei die Konzentration der Verbindung vom Typ der Flavyliumsalze im Bereich von 0,001 bis 5 Gew.-%, bezogen auf das Gesamtgewicht der Zusammensetzung, liegt.

**16.** Zusammensetzung nach einem der Ansprüche 1 bis 15, wobei das DHA in Mengenanteilen von 0,1 bis 10 Gew.-%, bezogen auf das Gesamtgewicht der Zusammensetzung, enthalten ist.

**17.** Zusammensetzung nach einem der Ansprüche 1 bis 16, die mindestens 5 Gew.-% eines oder mehrerer polyhydroxylierter Lösungsmittel, bezogen auf das Gewicht der Zusammensetzung, enthält.

**18.** Zusammensetzung nach Anspruch 17, wobei die polyhydroxylierten Lösungsmittel unter den Glykolen und Glykolethern ausgewählt sind.

**19.** Zusammensetzung nach Anspruch 17 oder 18, wobei die polyhydroxylierten Lösungsmittel unter Ethylenglykol, Propylenglykol, Butylenglykol, Dipropylenglykol oder Diethylenglykol und deren Gemischen ausgewählt sind.

**20.** Zusammensetzung nach einem der Ansprüche 17 bis 19, die ein Gemisch von mindestens drei verschiedenen polyhydroxylierten Lösungsmitteln enthält.

**21.** Zusammensetzung nach Anspruch 20, die ein Gemisch enthält, das aus Propylenglykol, Butylenglykol und Dipropylenglykol besteht.

**22.** Verfahren zur kosmetischen Behandlung der Haut, das dazu dient, der Haut eine künstliche Färbung zu geben, die der natürlichen Bräunung nahe kommt, **dadurch gekennzeichnet, dass** es darin besteht, auf die Haut eine wirksame Menge einer Zusammensetzung nach einem der Ansprüche 1 bis 21 aufzutragen.

**23.** Verwendung von Dihydroxyaceton (DHA) und mindestens einer Verbindung vom Typ der Flavyliumsalze nach einem der vorhergehenden Ansprüche in kosmetischen und/ oder dermatologischen Zusammensetzungen oder zu deren Herstellung, um der Haut eine künstliche Färbung zu geben, die der natürlichen Bräunung nahe kommt.

## Claims

**1.** Cosmetic and/or dermatological composition intended for giving the skin an artificial coloration close to that of a natural tan, **characterized in that** it comprises, in a cosmetically acceptable support, dihydroxyacetone (DHA)

and at least one flavylium salt compound which is not substituted in position 3 and which is substituted with at least one hydroxyl or alkoxy radical, which corresponds to formula (I) below:

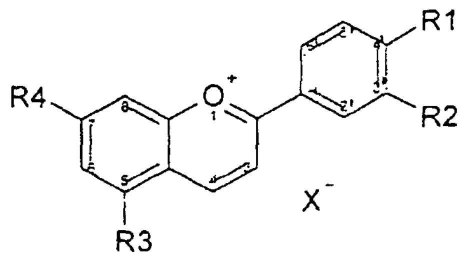

(I)

in which:

- $R_1$ denotes an OH or linear or branched, saturated or unsaturated $C_1$-$C_8$ alkoxy radical,
- $R_2$, $R_3$ and $R_4$, which may be identical or different, denote H or $R_1$, it being understood that at least one of the radicals $R_1$ to $R_4$ denotes OH,
- $X^-$ is an organic or mineral anion, and is obtained synthetically or from a plant extract containing it or alternatively from an enriched plant extract.

2. Composition according to Claim 1, **characterized in that** it leads, 30 minutes after application to a fair skin at a rate of 2 $mg/cm^2$, to a darkening of the colour of the skin **characterized in** the L* a* b* colorimetric measuring system by a $\Delta L^*$ ranging from -0.5 to -20.

3. Composition according to Claim 2, in which the $\Delta L^*$ ranges from -0.5 to -15.

4. Composition according to Claim 1 to 3, **characterized in that** it produces, 30 minutes after application to a fair skin at a rate of 2 $mg/cm^2$, a coloration defined in the L* a* b* colorimetric system by a ratio $\Delta a^*/\Delta b^*$ ranging from 0.5 to 3.

5. Composition according to Claim 4, according to which the ratio $\Delta a^*/\Delta b^*$ ranges from 0.8 to 2.

6. Composition according to Claim 1, in which, in formula (I) $X^-$ is a halide or an organic acid derivative.

7. Composition according to Claim 1, in which, in formula (I) $R_1$ denotes OH or $OCH_3$.

8. Composition according to Claim 1, in which the compound of formula (I) is chosen from the chlorides of the following compounds:

- 4',5,7-trihydroxyflavylium (apigeninidine chloride),
- 3',4',7-trihydroxyflavylium,
- 4'-hydroxyflavylium,
- 4',7-dihydroxyflavylium,
- 3',4'-dihydroxyflavylium,
- 3',4'-dihydroxy-7-methoxyflavylium,
- 3',4',5,7-tetrahydroxyflavylium,
- 3',4',5',5,7-pentahydroxyflavylium.

9. Composition according to Claim 8, **characterized in that** it is 4',5,7-trihydroxyflavylium chloride in pure form, which can be obtained synthetically.

10. Composition according to Claim 8, **characterized in that** it is 4',5,7-trihydroxyflavylium chloride, in the form of a plant extract.

11. Composition according to Claim 10, **characterized in that** the plant extract is a plant extract obtained from leaves of Sorghum caudatum; from stems, seeds or leaves of Sorghum bicolor; from the petals of Gesneria fulgens, and also from the species Blechum procerum and Sorghum in combination with Colletotrichum graminicola.

**12.** Composition according to Claim 10, **characterized in that** the plant extract is an extract of Sorghum bicolor which may be obtained by an acidic aqueous-alcoholic extraction at an extraction temperature ranging from 30°C to 40°C with a ratio of the volume of solvent to the mass of Sorghum bicolor leaves ranging from 10 to 30.

**13.** Composition according to Claim 12, **characterized in that** the extract of Sorghum bicolor has a titre of from 0.05% to 50% by weight of 4',5,7-trihydroxyflavylium chloride salt.

**14.** Composition according to any one of Claims 1 to 13, in which the concentration of flavylium salt compound ranges from 0.0001% to 10% relative to the total weight of the composition.

**15.** Composition according to Claim 14, in which the concentration of flavylium salt compound ranges from 0.001% to 5% by weight relative to the total weight of the composition.

**16.** Composition according to any one of Claims 1 to 15, in which dihydroxyacetone is present in proportions ranging from 0.1% to 10% by weight relative to the total weight of the composition

**17.** Composition according to any one of Claims 1 to 16, containing at least 5% by weight, relative to the weight of the composition, of one or more polyhydroxylated solvents.

**18.** Composition according to Claim 17, in which the polyhydroxylated solvents are chosen from glycols and glycol ethers.

**19.** Composition according to Claim 17 or 18, in which the polyhydroxylated solvents are chosen from ethylene glycol, propylene glycol, butylene glycol, dipropylene glycol and diethylene glycol, and mixtures thereof.

**20.** Composition according to any one of Claims 17 to 19, containing a mixture of three different polyhydroxylated solvents.

**21.** Composition according to Claim 20, containing a mixture consisting of propylene glycol, butylene glycol and dipropylene glycol.

**22.** Cosmetic treatment process for the skin which is intended to give it an artificial coloration close to that of a natural tan, **characterized in that** it consists in applying to the skin an effective amount of a composition as defined in any one of Claims 1 to 21.

**23.** Use of dihydroxyacetone (DHA) and at least one flavylium salt compound as defined in any one of the preceding claims, in or for the manufacture of cosmetic and/or dermatological compositions with the aim of giving the skin an artificial coloration close to that of a natural tan.